# EUROPEAN PATENT APPLICATION

(11) **EP 1 548 013 A1**
(43) Date of publication of application: **29.06.2005**
(21) Application number: 03748633.9
(22) Date of filing: 01.10.2003
(51) Int. Cl.: C07D 277/12, B01J 31/02, B01J 38/00, C07C 321/04, C07C 321/08

(54) **PROCESS FOR PRODUCING OPTICALLY ACTIVE ALPHA-SUBSTITUTED CYSTEINE OR SALT THEREOF, INTERMEDIATE THEREFOR, AND PROCESS FOR PRODUCING THE SAME**

(30) Priority: 01.10.2002 JP 2002288401; 25.07.2003 JP 2003201787
(71) Applicant: KANEKA CORPORATION, Osaka-shi, Osaka 530-8288 (JP)
(72) Inventor: MARUOKA, Keiji, Kyoto University, Kyoto-shi, Kyoto 606-8501 (JP); OOI, Takashi, Kyoto University, Kyoto-shi, Kyoto 606-8501 (JP); INOUE, Kenji, Kaneka Corp. Takasago Plant, Takasago-shi, Hyogo 676-8688 (JP)
(74) Representative: HOFFMANN - EITLE
(86) International application number: PCT/JP2003/012565
(87) International publication number: WO 2004/031163

(57) **Abstract**

The present invention provides a simple, practical, and industrially advantageous process for producing an optically active α-substituted cysteine or a salt thereof from inexpensive and readily available materials.

The present invention provides a process for producing an optically active α-substituted cysteine or a salt thereof by converting a cysteine derivative into a thiazoline compound and subjecting the resulting thiazoline compound to a stereoselective substituent-introducing reaction catalyzed by an optically active quaternary ammonium salt, in particular, an axially asymmetric quaternary ammonium salt to produce an optically active thiazoline compound and then hydrolyzing the resulting thiazoline compound.

## Description

### Technical Field

The present invention relates to a process for producing an optically active α-substituted cysteine or a salt thereof, which is useful as an intermediate for, for example, pharmaceuticals, and also relates to an intermediate useful for synthesizing the optically active α-substituted cysteine or a salt thereof and a process for producing the intermediate.

### Background Art

The following methods for producing optically active α-substituted cysteine derivatives or salts thereof have been known, the derivatives being one of optically active amino acid derivatives each having two different substituents at the α position:
(1) a method for asymmetrically alkylating an optically active thiazolidine compound prepared from an optically active cysteine and pivalaldehyde (PCT Japanese Translation Patent Publication No. 2000-515166, WO01/72702, and WO01/72703);
(2) a method for asymmetrically thioalkylating an optically active thiazolidine compound prepared from an optically active alanine and benzaldehyde (Tetrahedron, 1999, 55, 10685-10694);
(3) a method for asymmetrically bromomethylating an optically active diketopiperazine compound prepared from an optically active valine and alanine and then replacing the bromine atom of the resulting compound with an alkali metal alkylthiolate (J. Org. Chem., 1992, 57, 5568-5573);
(4) a method for synthesizing an optically active aziridine compound from an optically active 2-methyl glycidol prepared by Sharpless asymmetric oxidation of 2-methyl-2-propen-1-ol and then for allowing the resulting aziridine compound to react with thiol (J. Org. Chem., 1995, 60, 790-791);
(5) a method for alkylating an aminomalonic acid derivative and performing asymmetrization with a pig liver esterase (hereinafter, referred to as "PLE") and then allowing the resulting optically active ester to react with an alkali metal thioacetate (J. Am. Chem. Soc., 1993, 115, 8449-8450); and
(6) a method for resolving and purifying a racemic thiazoline compound by chiral high performance liquid chromatography (HPLC), the racemic thiazoline compound being synthesized by methylating a thiazoline compound prepared from a cysteine derivative (Synlett., 1994, 9, 702-704).

However, any one of the methods (1), (2), and (3) includes a low temperature reaction with an expensive base such as butyl lithium and thus requires a special production facility. The method (4) includes many steps and is thus complicated; hence, the method (4) is industrially disadvantageous. In the method (5), the asymmetrization of a diester with the PLE is a key step. It is difficult to mass-produce the PLE and thus to ensure an industrial-scale stable supply of the PLE; hence, the method (5) is impractical. In the method (6), since the asymmetric alkylation of a thiazoline compound has not been known, it is necessary to resolve the racemic mixture by the chiral HPLC. However, since the resolved needless enantiomer cannot be reused by racemization, productivity is low; thus this method is disadvantageous for mass production.

As described above, any method for industrially producing an optically active α-substituted cysteine or a salt thereof has problems to be solved.

### Disclosure of Invention

In view of the above-described problems, it is an object of the present invention to provide a practical and industrially advantageous process for producing an optically active α-substituted cysteine or a salt thereof from inexpensive and readily available materials.

In view of the above-described circumstances, the present inventors have conducted intensive studies and found a process for producing an optically active α-substituted cysteine or a salt thereof by converting a cysteine derivative into a thiazoline compound, subjecting the resulting thiazoline compound to a stereoselective substituent-introducing reaction with an optically active quaternary ammonium salt, particularly, an optically active axially asymmetric quaternary ammonium salt functioning as a catalyst to produce an optically active thiazoline compound, and then hydrolyzing the resulting thiazoline compound. The finding has led to the completion of the present invention.

That is, the present invention relates to a process for producing an optically active thiazoline compound represented by general formula (1): 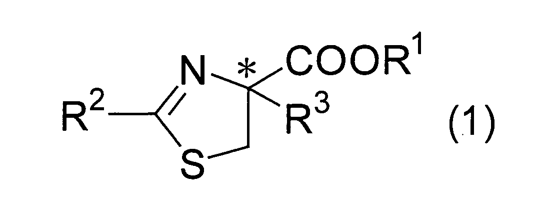 (where * represents an asymmetric carbon atom; R¹ represents an optionally substituted linear, branched, or cyclic C₁-C₁₀ alkyl group or an optionally substituted linear, branched, or cyclic C₁-C₁₀ alkylsilyl group; R² represents an optionally substituted C₆-C₃₀ aryl group or an optionally substituted linear, branched, or cyclic C₁-C₂₀ alkyl group; and R³ represents an optionally substituted linear, branched, or cyclic C₁-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group), the process including a step of allowing a thiazoline compound represented by general formula (2): 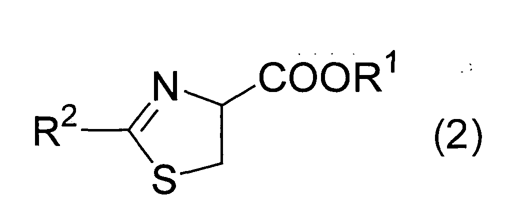 (where R¹ and R² are the same as above) to react with a compound represented by general formula (3) in the presence of a base and an optically active quaternary ammonium salt functioning as a catalyst:

R³L (3)

(R³ is the same as above; and L represents a leaving group).

The present invention also relates to a process for producing an optically active α-substituted cysteine represented by general formula (6) or a salt thereof: 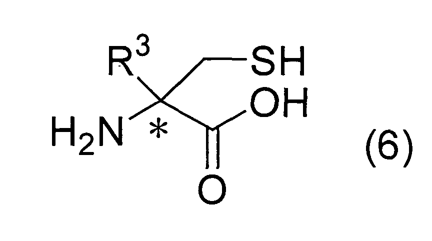 (where * and R³ are the same as above), the process including a step of hydrolyzing an optically active thiazoline compound represented by general formula (1): 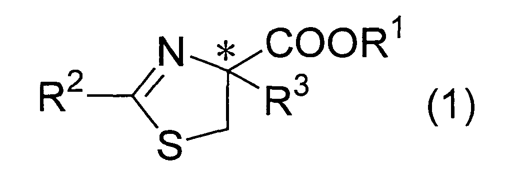 (where *, R¹, R², and R³ are the same as above).

The present invention further relates to an optically active thiazoline compound represented by general formula (7) : 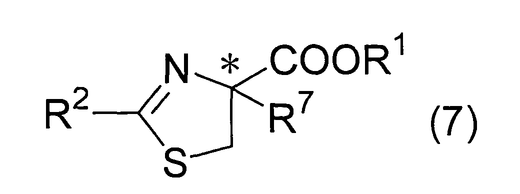 (where *, R¹, and R² are the same as above; R⁷ represents an optionally substituted linear, branched, or cyclic C₂-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group).

The present invention will be described in detail below.

The present invention provides a process for producing an optically active thiazoline compound represented by general formula (1): 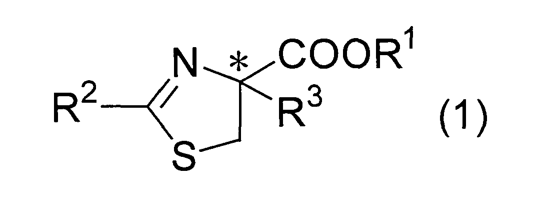 , the process including a step of allowing a thiazoline compound represented by general formula (2): 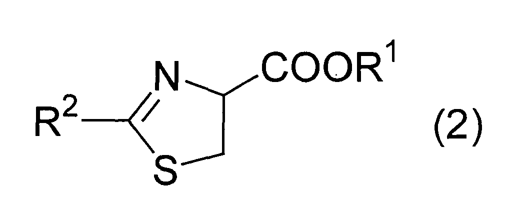 to react with a compound represented by general formula (3) in the presence of a base and an optically active quaternary ammonium salt functioning as a catalyst:

R³L (3)

The thiazoline compound represented by general formula (2) will now be described.

In general formula (2), R¹ represents an optionally substituted linear, branched, or cyclic C₁-C₁₀ alkyl group, or an optionally substituted linear, branched, or cyclic C₁-C₁₀ alkylsilyl group; and R² represents an optionally substituted C₆-C₃₀ aryl group or an optionally substituted linear, branched, or cyclic C₁-C₂₀ alkyl group. The number of carbon atoms in each of R¹ and R² excludes the number of carbon atoms in the substituent. Examples of the substituent in R¹ include halogen atoms (a fluorine atom, a chlorine atom, a bromine atom, and an iodine atom). Examples of the substituent in R² include halogen atoms (a fluorine atom, a chlorine atom, a bromine atom, and an iodine atom); and an alkoxyl group (such as a methoxy group and an ethoxy group).

Examples of the optionally substituted linear, branched, or cyclic C₁-C₁₀ alkyl group as R¹ include a methyl group, a trifluoromethyl group, an ethyl group, an *n*-propyl group, an isopropyl group, an *n*-butyl group, a *sec*-butyl group, and a *tert*-butyl group.

Examples of the optionally substituted linear, branched, or cyclic C₁-C₁₀ alkylsilyl group as R¹ include a tertbutyldimethylsilyl group and a trimethylsilyl group.

To ensure high selectivity, R¹ is preferably an optionally substituted linear, branched, or cyclic C₁-C₁₀ alkyl group, more preferably a methyl group, an ethyl group, an *n*-propyl group, an isopropyl group, an *n*-butyl group, a *sec*-butyl group, or a *tert*-butyl group, and most preferably an ethyl group or a *tert*-butyl group.

Examples of the optionally substituted C₆-C₃₀ aryl group as R² include a phenyl group, a 2-methoxyphenyl group, a 3-methoxyphenyl group, a 4-methoxyphenyl group, a naphthyl group, an anthryl group, a phenanthryl group, and a terphenyl group.

Examples of the optionally substituted linear, branched, or cyclic C₁-C₂₀ alkyl group as R² include a methyl group, a trifluoromethyl group, a trichloromethyl group, an ethyl group, an *n*-propyl group, an isopropyl group, a cyclopropyl group, an *n*-butyl group, a *sec*-butyl group, a *tert*-butyl group, a cyclobutyl group, a pentyl group, a cyclopentyl group, a hexyl group, and a cyclohexyl group.

From the standpoints of ease of synthesis and high selectivity in the stereoselective substituent-introducing reaction, R² is preferably an optionally substituted phenyl group and more preferably a phenyl group or a 4-methoxyphenyl group.

The thiazoline compound represented by general formula (2) can be prepared by direct reaction between a nitrile compound and a cysteine derivative or by treating a nitrile compound with hydrogen chloride to produce an imidate and then allowing the resulting imidate to react with a cysteine derivative, as reported in, for example, Synlett, 1994, 9, 702-704. The nitrile compound is not particularly limited as long as the nitrile compound is available.

Next, the compound represented by general formula (3) will be described.

In general formula (3), R³ represents an optionally substituted linear, branched, or cyclic C₁-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group. L represents a leaving group. The number of carbon atoms in R³ excludes the number of carbon atoms in the substituent. Examples of the substituent in R³ include halogen atoms (a fluorine atom, a chlorine atom, a bromine atom, and an iodine atom); alkoxyl groups (such as a methoxy group, an ethoxy group, a propoxy group, and a butoxy group); and alkyl groups (such as a methyl group and an ethyl group).

The compound represented by general formula (3) is not particularly limited as long as the compound can be allowed to react with the thiazoline compound represented by general formula (2).

Examples of the optionally substituted linear, branched, or cyclic C₁-C₂₀ alkyl group as R³ include a methyl group, an ethyl group, an *n*-propyl group, an isopropyl group, an *n*-butyl group, a *sec*-butyl group, a *tert*-butyl group, a pentyl group, a hexyl group, a cyclopropylmethyl group, a cyclopentylmethyl group, and a cyclohexylmethyl group.

Examples of the optionally substituted linear, branched, or cyclic C₂-C₂₀ alkenyl group include an allyl group, a 2-butenyl group, a 1-methyl-2-propenyl group, and 2-methyl-2-propenyl group.

Examples of the optionally substituted linear, branched, or cyclic C₂-C₂₀ alkynyl group include an ethynyl group, a 1-propynyl group, a propargyl group, a butynyl group, a pentynyl group, and a hexynyl group.

Examples of the optionally substituted linear, branched, or cyclic C₃-C₂₀ alkoxycarbonylalkyl group include a *tert*-butoxycarbonylmethyl group, and a *tert*-butoxycarbonylethyl group.

Examples of the optionally substituted C₇-C₃₀ aralkyl group include a benzyl group, a chlorobenzyl group, a fluorobenzyl group, a bromobenzyl group, a dichlorobenzyl group, a difluorobenzyl group, a dibromobenzyl group, a methylbenzyl group, a methoxybenzyl group, a 3,4-dibutoxybenzyl group, and a naphthylmethyl group.

Examples of the optionally substituted C₄-C₃₀ heteroaralkyl group include a pyridylmethyl group, a difluoropyridylmethyl group, a quinolylmethyl group, an indolylmethyl group, a furfuryl group, and a thienylmethyl group.

In the present invention, R³ is preferably an optionally substituted linear, branched, or cyclic C₁-C₂₀ alkyl group, an optionally substituted linear, branched, or cyclic C₂-C₂₀ alkenyl group, an optionally substituted linear, branched, or cyclic C₂-C₂₀ alkynyl group, or an optionally substituted C₇-C₃₀ aralkyl group, and more preferably a methyl group, an ethyl group, an allyl group, a propargyl group, or a benzyl group.

Examples of the leaving group L include halogen atoms (a fluorine atom, a chlorine atom, a bromine atom, and an iodine atom); and a methanesulfonyloxy group, a *p*-toluenesulfonyloxy group, and a trifluoromethanesulfonyloxy group. The halogen atoms are preferable. Among the halogen atoms, a chlorine atom, an iodine atom, or bromine atom is particularly preferable.

Next, the optically active quaternary ammonium salt will be described.

The optically active quaternary ammonium salt used in the process of the present invention is not particularly limited as long as it is generally used as, for example, a phase-transfer catalyst. Examples of the optically active quaternary ammonium salt include *N*-benzylcinchoninium chloride, *N*-benzylcinchoninium bromide, N-benzylcinchonidinium chloride, *N*-benzylcinchonidinium bromide, *N*-*p*-trifluoromethylbenzylcinchoninium chloride, *N-N*-*p*-trifluoromethylbenzylcinchoninium bromide, N-*N*-*p*-trifluoromethylbenzylcinchonidinium chloride, *N-N-p*-trifluoromethylbenzylcinchonidinium bromide, and optically active axially asymmetric quaternary ammonium salts represented by general formulae (4) and (5): 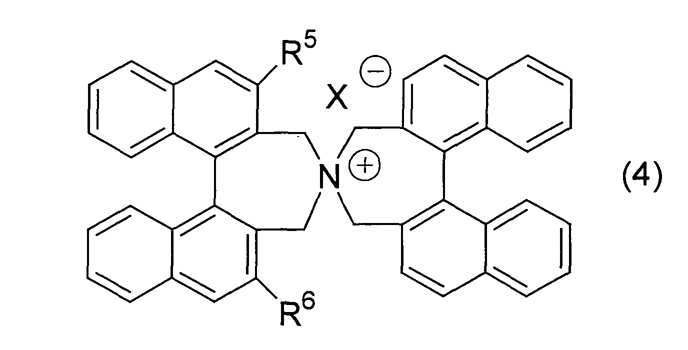 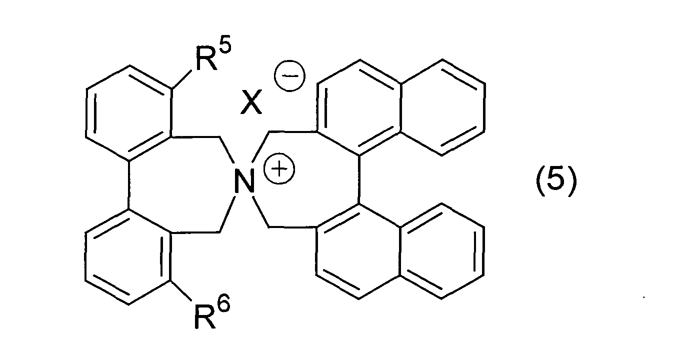 (where R⁵ and R⁶ are described below).

The optically active quaternary ammonium salt is preferably an optically active axially asymmetric quaternary ammonium salt represented by formula (4) or (5).

The term "optical activity" means that the content of a specific optical isomer among potential optical isomers is higher than that of the other optical isomers. In the present invention, when an optically active axially asymmetric quaternary ammonium salt represented by formula (4) or (5) is used as a catalyst for the reaction for stereoselectively introducing a substituent into the thiazoline compound (2), the content of a specific optical isomer is preferably at least 90%, more preferably at least 95%, and still more preferably at least 98% in order to achieve a higher stereoselectivity.

In general formulae (4) and (5), R⁵ and R⁶ each represent a hydrogen atom; an optionally substituted linear, branched, or cyclic C₁-C₂₀ alkyl group; an optionally substituted linear, branched, or cyclic C₂-C₂₀ alkenyl group; an optionally substituted linear, branched, or cyclic C₂-C₂₀ alkynyl group; an optionally substituted C₆-C₃₀ aryl group; an optionally substituted C₃-C₃ₒ heteroaryl group; an optionally substituted C₇-C₃₀ aralkyl group; an optionally substituted C₄-C₃₀ heteroaralkyl group; an optionally substituted linear, branched, or cyclic C₁-C₁₅ alkanoyl group; or a C₇-C₃₀ aroyl group having an optionally substituted aromatic ring. R⁵ and R⁶ may be the same or different. The number of carbon atoms in each of R⁵ and R⁶ excludes the number of carbon atoms in the substituents. Examples of the substituents in R⁵ and R⁶ include halogen atoms (a fluorine atom, a chlorine atom, a bromine atom, and an iodine atom), alkoxy groups (such as a methoxy group, an ethoxy group, a propoxy group, and a butoxy group), aryl groups (such as a phenyl group and a naphthyl group), cycloalkyl groups (such as a cyclopropyl group and a cyclobutyl group), alkyl groups (such as a methyl group, an ethyl group, a propyl group, and a butyl group), hydroxyl groups, and amino groups.

Examples of the optionally substituted linear, branched, or cyclic C₁-C₂₀ alkyl groups as R⁵ and R⁶ include a methyl group, a trifluoromethyl group, a *tert*-butoxymethyl group, an ethyl group, an *n*-propyl group, an isopropyl group, a cyclopropyl group, an *n*-butyl group, a *sec*-butyl group, a *tert*-butyl group, a cyclobutyl group, a pentyl group, a cyclopentyl group, a hexyl group, and a cyclohexyl group.

Examples of the optionally substituted linear, branched, or cyclic C₂-C₂₀ alkenyl group include a vinyl group, a 1-propenyl group, an allyl group, an isopropenyl group, a cyclopropenyl group, a butenyl group, a cyclobutenyl group, a pentenyl group, a cyclopentenyl group, a hexenyl group, a cyclohexenyl group, and a styryl group.

Examples of the optionally substituted linear, branched, or cyclic C₂-C₂₀ alkynyl group include an ethynyl group, a phenylethynyl group, a cyclopropylethynyl group, a cyclobutylethynyl group, a 1-propynyl group, a propargyl group, a butynyl group, a pentynyl group, and a hexynyl group.

Examples of the optionally substituted C₆-C₃₀ aryl group include a phenyl group, a 3,4,5-trifluorophenyl group, a 3,5-*tert*-butylphenyl group, a naphthyl group, an anthryl group, a phenanthryl group, a terphenyl group, and a 3',3'',5',5''-*tert*-butyl-*m*-terphenyl group.

Examples of the optionally substituted C₃-C₃₀ heteroaryl group include a pyrrolinyl group, a pyridyl group, a quinolyl group, an imidazolyl group, a furyl group, an indolyl group, a thienyl group, an oxazolyl group, a thiazolyl group, a 2-phenylthiazolyl group, and an 2-anisylthiazolyl group.

Examples of the optionally substituted C₇-C₃₀ aralkyl group include a benzyl group, a chlorobenzyl group, a bromobenzyl group, a phenethyl group, a naphthylmethyl group, an anthracenylmethyl group, a 3,5-difluorobenzyl group, and a trityl group.

Examples of the optionally substituted C₄-C₃₀ heteroaralkyl group include a pyridylmethyl group, a difluoropyridylmethyl group, a quinolylmethyl group, an indolylmethyl group, a furfuryl group, and a thienylmethyl group.

Examples of the optionally substituted linear, branched, or cyclic C₁-C₁₅ alkanoyl group include an acetyl group, a propanoyl group, a butyloyl group, a pentanoyl group, a cyclopentanecarbonyl group, a hexanoyl group, a cyclohexanecarbonyl group, a glycoloyl group, a glycyl group, and a cinnamoyl group.

Examples of the C₇-C₃₀ aroyl group having an optionally substituted aromatic ring include a benzoyl group, a salicyloyl group, and a naphthoyl group.

In the present invention, to achieve a higher yield and selectivity, R⁵ and R⁶ each are preferably an optionally substituted aryl group, more preferably an optionally substituted phenyl group, an optionally substituted naphthyl group, an optionally substituted anthryl group, an optionally substituted phenanthryl group, or an optionally substituted terphenyl group, still more preferably a phenyl group, a 3,4,5-trifluorophenyl group, a 3,5-*tert*-butylphenyl group, a naphthyl group, an anthryl group, a phenanthryl group, a terphenyl group, or a 3',3",5',5"-*tert*-butyl-*m*-terphenyl group, and most preferably a naphthyl group, a 3,4,5-trifluorophenyl group, a 3,5-*tert*-butylphenyl group, or a 3',3",5',5"-*tert*-butyl-*m*-terphenyl group.

In formulae (4) and (5), R⁵ and R⁶ are preferably the same group.

Each X in formulae (4) and (5) represents a hetero atom or an atomic group having ability to function as a counter anion to the ammonium cation. Examples of the X include halogen atoms such as a fluorine atom, a chlorine atom, a bromine atom, and an iodine atom; and a hexafluorophosphate anion, a trifluoromethylsulfonate anion, and a methylsulfonate anion.

In the present invention, to achieve a higher yield and selectivity, X is preferably a halogen atom and more preferably a bromine atom.

The optically active axially asymmetric quaternary ammonium salt represented by formula (4) or (5) is prepared by a method disclosed in, for example, J. Am. Chem. Soc., 1999, 121, 6515-6520, J. Am. Chem. Soc., 2000, 122, 5228-5229, or Japanese Unexamined Patent Application Publication No. 2001-48866.

Next, the reaction for stereoselectively introducing a substituent into the thiazoline compound (2) with an optically active quaternary ammonium salt, particularly an optically active axially asymmetric quaternary ammonium salt functioning as a catalyst will be described.

The reaction is usually performed in a solvent. An organic solvent alone or a two-phase solvent system containing an organic solvent and water may be used as the reaction solvent. The organic solvent used for the reaction in the organic solvent alone or the two-phase solvent system containing the organic solvent and water is not particularly limited as long as the thiazoline compound (2) as a substrate, the compound (3), and the catalyst can partially or completely dissolve in the organic solvent. Examples of the organic solvent include benzene, toluene, xylenes, diethyl ether, isopropyl ether, tetrahydrofuran, acetonitrile, dioxane, methylene chloride, chloroform, and ethyl acetate. Use of toluene achieves high yield and high selectivity, and toluene is industrially advantageous due to its inexpensiveness; hence, toluene is preferably used.

The volume of the solvent is preferably adjusted such that the ratio of the volume (mL) of the solvent to the weight (g) of the thiazoline compound (2), i.e., the ratio mL/g, is 5 to 60 and more preferably 6 to 40.

When the two-phase solvent system containing an organic solvent and water is used, the mixing ratio of the organic solvent to water is not particularly limited.

The reaction is performed in the presence of a base. The base is not particularly limited as long as the base can generate the enolate of the thiazoline compound (2).

When the reaction is performed in the organic solvent alone, examples of the base include organic bases such as butyl lithium and lithium diisopropylamine; and inorganic bases such as sodium hydride and cesium hydroxide monohydrate. To achieve high selectivity, cesium hydroxide monohydrate is preferably used.

When the reaction is performed in the two-phase solvent system containing an organic solvent and water, an inorganic base can be used. Examples of the inorganic base include hydroxides such as lithium hydroxide, sodium hydroxide, potassium hydroxide, rubidium hydroxide, and cesium hydroxide. Use of inexpensive sodium hydroxide or potassium hydroxide in the reaction results in excellent yield and selectivity and is thus preferable.

The amount of the base used is preferably 1 to 50 equivalents and more preferably 1 to 20 equivalents, based on the thiazoline compound (2). The inorganic base is used in the form of an aqueous solution. The aqueous solution has an inorganic-base content of preferably 10 to 80% by weight and more preferably 30 to 60% by weight.

The optically active axially asymmetric quaternary ammonium salt represented by general formula (4) or (5) functions as a chiral catalyst in the reaction for stereoselectively introducing a substituent into the thiazoline compound (2). Since the catalyst represented by general formula (4) has two axial asymmetries, four isomers are present. In general, a catalyst in which the two axial asymmetries have the same configuration tends to provide higher selectivity.

The stereochemical configuration of the product prepared with the optically active axially asymmetric quaternary ammonium salt represented by general formula (4) or (5) varies depending on the stereochemical configuration of the salt. That is, use of the catalysts which are a pair of enantiomers results in respective products having different stereochemical configurations from each other with the same selectivity and yield. Therefore, selecting the catalyst used can control the stereochemical configuration of a target product.

The amount of optically active quaternary ammonium salt used as a catalyst is preferably 0.01 to 20 mol% and more preferably 0.1 to 1 mol%, based on the number of moles of the thiazoline compound (2).

The amount of compound (3) used is preferably 1 to 10 equivalents and more preferably 1 to 5 equivalents, based on the thiazoline compound (2).

In general, the above-described reaction can be performed by adding the base, the thiazoline compound (2), the compound (3), and the catalyst to the solvent in air or an inert gas. For the reaction in air, in some thiazoline compounds, the oxidation reaction of the thiazoline compound preferentially occurs rather than the stereoselective substituent-introducing reaction to form a thiazole. As a result, a target optically active thiazoline compound can hardly be produced. Thus, the reaction is preferably performed in an inert gas.

For the reaction in a two-phase system containing water and an organic solvent, the reaction temperature is preferably 0°C to 50°C and more preferably 0°C to 10°C. For the reaction in an organic solvent alone, the reaction temperature is preferably -20°C to 50°C and more preferably -20°C to 10°C.

The reaction time is preferably about 0.5 to 24 hours. A longer reaction time may partially oxidize the thiazoline compound (2) into thiazole. Thus, the reaction time is more preferably about 0.5 to 12 hours.

With respect to post-treatment of the reaction, for example, water is added to the resulting solution, and then a reaction product, that is, an optically active thiazoline compound (1) is extracted with an appropriate organic solvent. The extracting solvent is not particularly limited as long as the reaction product can dissolve in the solvent. General organic solvents such as diethyl ether, ethyl acetate, and toluene can be used.

The organic layer resulted from the extraction is then dried over and concentrated to produce a crude product. The crude product may be directly subjected to a next ring-opening reaction of the thiazoline ring. Alternatively, the crude product may be purified. The optically active thiazoline compound (1) can be isolated and purified by silica-gel column chromatography.

Furthermore, in this reaction, the chiral catalyst can be recovered and reused. That is, after the reaction, it is preferable that the optically active axially asymmetric quaternary ammonium salt represented by formula (4) or (5) be separated and recovered from the reaction mixture by column chromatography using a column packed with an adsorbent, and then the recovered salt be reused.

For example, after the reaction, the reaction solution is diluted with water and neutralized with an acid. Extraction is performed with an appropriate organic solvent, and then the resulting organic layer is dried over and concentrated to produce a crude product. This crude product is separated into the optically active thiazoline compound (1) and the chiral catalyst by column chromatography using a column packed with an appropriate adsorbent. In this way, the catalyst can be recovered.

Examples of the acid used for the neutralization include hydrochloric acid, sulfuric acid, acetic acid, hydrobromic acid, and trifluoroacetic acid. Hydrobromic acid is preferably used in order that the catalyst recovered can have high reaction activity and selectivity.

The extracting solvent is not particularly limited as long as the reaction product and the catalyst can dissolve in the extracting solvent, and general organic solvents can be used. For example, ethyl acetate, methylene chloride, chloroform, diethyl ether, or toluene can be used. Ethyl acetate or methylene chloride is preferable.

The adsorbent with which the column is packed is not particularly limited but is preferably alumina, silica gel, or the like. Silica gel is more preferable from the standpoint of separability.

With respect to an eluent used in column chromatography, an appropriate combination of organic solvents depending on a target product may be used in order to elute the optically active thiazoline compound (1). Examples of the organic solvent include ethyl acetate, hexane, chloroform, methylene chloride, methanol, ethanol, and diethyl ether. A combination of organic solvents containing at least one selected from the group consisting of these may be used.

After the elution of the optically active thiazoline (1), the catalyst can be recovered with an eluent having a higher polarity. A mixed solvent system containing a low-polarity organic solvent and an alcohol at an appropriate ratio is used as the eluent having a higher polarity. Examples of the low-polarity organic solvent include chloroform, methylene chloride, ethyl acetate, and hexane. Methylene chloride is preferable. Examples of the alcohol include methanol, ethanol, *n*-propanol, and isopropanol. Methanol is preferable due to ease of the removal of the solvent by distillation and the efficiency of elution.

The mixing ratio of the low-polarity organic solvent to the alcohol may be adjusted depending on the type of the catalyst to be recovered. The volume of methylene chloride used is preferably 5 to 50 times and more preferably 10 to 30 times that of methanol.

The recovered catalyst can be reused directly without any treating.

The present invention also provides a process for producing an optically active α-substituted cysteine represented by general formula (6) or a salt thereof: 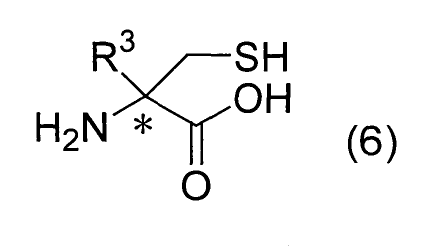 (where * and R³ are the same as above), the process including a step of hydrolyzing an optically active thiazoline compound represented by general formula (1): 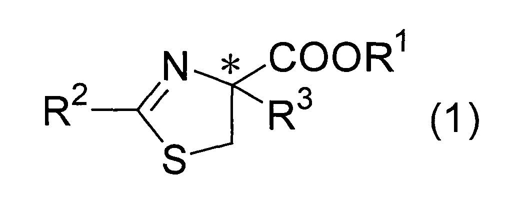 (where *, R¹, R², and R³ are the same as above). That is, by hydrolyzing the resulting optically active thiazoline compound (1), an optically active α-substituted cysteine (6) or a salt thereof can be produced.

The process for the hydrolysis is not particularly limited as long as the process can open the thiazoline ring. An example of the process includes an approach with an acid or an alkali.

Examples of the acid used include hydrochloric acid, acetic acid, sulfuric acid, hydrobromic acid, and trifluoroacetic acid. Hydrochloric acid is preferable.

Examples of the alkali used include hydroxides such as lithium hydroxide, sodium hydroxide, potassium hydroxide, calcium hydroxide, barium hydroxide, and magnesium hydroxide. Lithium hydroxide, sodium hydroxide, or potassium hydroxide is preferable.

The acid and the base each are preferably used in the form of an aqueous solution. The concentration is preferably 0.1 to 20 N and more preferably 0.1 to 10 N.

The hydrolysis is preferably performed with the acid.

The reaction solvent may be the acidic aqueous solution or alkaline aqueous solution used for the hydrolysis. Alternatively, the reaction solvent may be a mixture of water and a solvent such as toluene, acetonitrile, tetrahydrofuran, methanol, or ethanol. The amounts of water and the solvent are not particularly limited.

The reaction temperature should be set at a temperature at which the reaction efficiently proceeds. The reaction temperature is preferably set at 70°C to 150°C and more preferably 90°C to 120°C.

With respect to the reaction time, the reaction is often completed in 12 to 24 hours. The disappearance of the optically active thiazoline compound (1) is confirmed by thin-layer chromatography (TLC). For a substrate being slow to react, a longer reaction time is required. By performing the reaction at high-temperature and high-pressure in a pressure-proof reactor, the reaction time can be reduced.

After the reaction, the resulting optically active α-substituted cysteine (6) or a salt thereof can be isolated and purified in an appropriate solvent by crystallization. The solvent used and the crystallization conditions should be each optimally selected for the resulting optically active α-substituted cysteine (6). The case of an optically active α-methyl cysteine hydrochloride will be described, the α-methyl cysteine hydrochloride being produced by methylating a thiazoline compound (2) to produce an optically active thiazoline compound (1) and then hydrolyzing the resulting compound (1) with hydrochloric acid.

After the reaction, the reaction mixture is concentrated under a reduced pressure until the total amount of hydrochloric acid is reduced to about 1/6. Then, toluene is added, and water is further removed azeotropically. During the step of repeating further addition of toluene and azeotropic removal of water, an optically active α-methyl cysteine hydrochloride is deposited as crystals. The resulting crystal is filtrated and washed with toluene, followed by drying to produce an optically active α-methyl cysteine hydrochloride with excellent purity and yield.

The present invention also provides an optically active thiazoline compound represented by general formula (7): 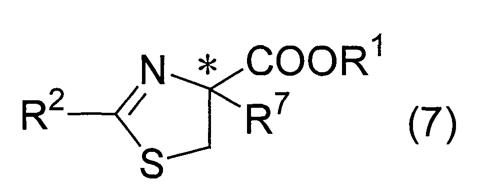 (where *, R¹, and R² are the same as above; R⁷ represents an optionally substituted linear, branched, or cyclic C₂-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group).

The optically active thiazoline compound (7) prepared in the present invention is a novel compound and can be an intermediate useful for the production of an optically active α-substituted cysteine or a salt thereof.

Groups represented by R¹ and R² have been described above.

Examples of the groups represented by R⁷ include the groups for R³ described above except for a methyl group. R⁷ represents preferably an ethyl group, an *n*-propyl group, an isopropyl group, an *n*-butyl group, a *sec*-butyl group, a *tert*-butyl group, a pentyl group, a hexyl group, a cyclopropylmethyl group, a cyclopentylmethyl group, a cyclohexylmethyl group, an allyl group, a 2-butenyl group, a 1-methyl-2-propenyl group, a 2-methyl-2-propenyl group, a propargyl group, a *tert*-butoxycarbonylmethyl group, a benzyl group, a chlorobenzyl group, a fluorobenzyl group, a bromobenzyl group, a dichlorobenzyl group, a difluorobenzyl group, a dibromobenzyl group, a methylbenzyl group, a methoxybenzyl group, a 3,4-dibutoxybenzyl group, a naphthylmethyl group, or an indolylmethyl group. An ethyl group, an allyl group, a propargyl group, or a benzyl group is more preferable.

### Best Mode for Carrying Out the Invention

The present invention will be described in detail with reference to examples below, but the present invention is not limited to these examples.

### (REFERENCE EXAMPLE 1) Process for producing ethyl 2-phenylthiazoline-4-carboxylate

Ethylbenzimidate hydrochloride (742.4 mg, 4 mmol), cysteine ethyl ester hydrochloride (779.9 mg, 4.2 mmol), and triethylamine (585.5 µl, 4.2 mmol) were dissolved in methanol (8 mL) and the resulting mixture was stirred at room temperature overnight. After addition of water, methanol was distilled off under a reduced pressure, and the remaining water layer was extracted with ethyl acetate. The resulting organic layer was dried over anhydrous sodium sulfate and concentrated under a reduced pressure to produce a crude product. The resulting product was purified by silica-gel column chromatography (hexane:ethyl acetate = 8:1) to yield target ethyl 2-phenylthiazoline-4-carboxylate (941.2 mg, yield: 99%).

### (REFERENCE EXAMPLE 2) Process for producing tert-butyl 2-phenylthiazoline-4-carboxylate

A 2 M sodium hydroxide aqueous solution (4 mL) was added to a methanol solution (4 mL) containing ethyl 2-phenylthiazoline-4-carboxylate (941.32 mg, 4 mmol) and the resulting mixture was stirred at room temperature for 30 minutes. After removal of methanol by distillation under reduced pressure, citric acid was added to the aqueous layer to make the solution acidic, and then extraction was performed with ethyl acetate. The resulting organic layer was dried over anhydrous sodium sulfate and concentrated under a reduced pressure to produce 2-phenylthiazoline-4-carboxylic acid. This was cooled to 0°C. A methylene chloride solution (8 mL) containing dimethylaminopyridine (391.0 mg, 3.2 mmol) and *tert*-butanol (1.15 mL, 12 mmol) was added, finally 1,3-dicyclohexylcarbodiimide (907.7 mg, 4.4 mmol) was added, and then the resulting mixture was stirred for 30 minutes. A generated urea compound was filtered off. The resulting filtrate was washed with 1 N hydrochloric acid, and then extraction was performed with ether. The resulting organic layer was dried over anhydrous sodium sulfate. Then, the solvent was distilled off under a reduced pressure to yield a crude product. The crude product was purified by silica-gel chromatography (hexane:ethyl acetate = 8:1) to yield target *tert*-butyl 2-phenylthiazoline-4-carboxylate (colorless oil; 547.9 mg, yield: 52%).
¹H NMR (400 MHz, CDCl₃) δ: 7.86-7.88 (2H, m), 7.39-7.49 (3H, m), 5.20 (1H, t), 3.63 (2H, d), 1.52 (9H, s).

### (EXAMPLE 1) Process for producing tert-butyl (R)-4-methyl-2-phenylthiazoline-4-carboxylate

In an argon atmosphere, toluene (2 mL) was added to *tert*-butyl 2-phenylthiazoline-4-carboxylate (79.0 mg, 0.3 mmol) prepared in REFERENCE EXAMPLE 2 and a catalyst (2.74 mg, 3 µmol) having the (*S,S*) configuration of two axial asymmetries and represented by general formula (8). Methyl iodide (37.3 µl, 0.6 mmol) was added, and the resulting mixture was cooled to 0°C. An aqueous solution of 50% sodium hydroxide (1 mL) was added, and the resulting mixture was stirred until the disappearance of *tert*-butyl 2-phenylthiazoline-4-carboxylate was confirmed by TLC.

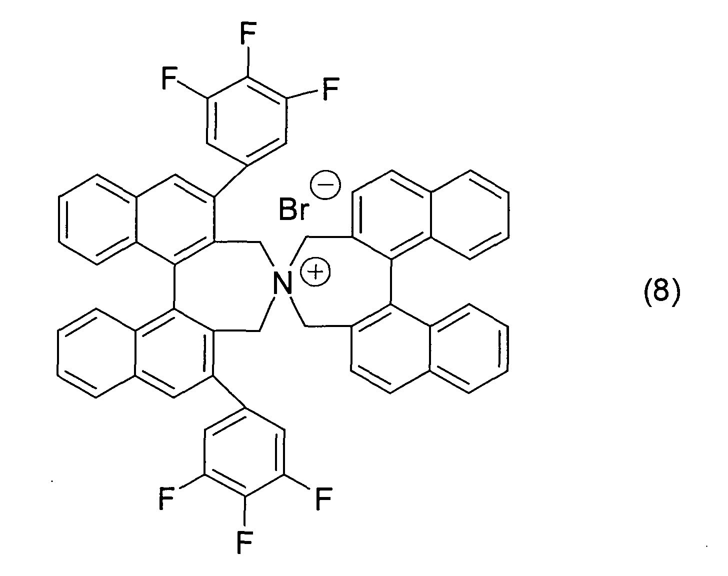

After the reaction, the mixture was diluted with water, and then extraction was performed with diethyl ether. The resulting organic layer was dried over anhydrous sodium sulfate and concentrated under a reduced pressure to yield a crude product. The crude product was purified by silica-gel column chromatography (hexane:diethyl ether = 10:1). As a result of ¹H NMR analysis of the resulting product, the product was identified as target optically active *tert*-butyl 4-methyl-2-phenylthiazoline-4-carboxylate (71.3 mg, yield: 86%). Analysis by HPLC (column: CHRALCEL OD (manufactured by Daicel Chemical Industries, Ltd.), mobile phase: hexane:ethanol = 400:1, retention time: 16.7 minutes (major), 20.8 minutes (minor)) showed that the optical purity was 97% e.e.

The retention time for a *tert*-butyl (*R*)-4-methyl-2-phenylthiazoline-4-carboxylate standard separately prepared with optically active (*R*)-methyl-L-cysteine *tert*-butyl ester as a starting material was 16.7 minutes. Thus, it was determined that the main product had the *R* configuration.
¹H NMR (400 MHz, CDCl₃) δ: 7.85-7.87 (2H, m), 7.40-7.47 (3H, m), 3.83 (1H, d), 3.25 (1H, d), 1.63 (3H, s), 1.50 (9H, s); [α]^{D}₂₇ = -17.5 (c 0.50, CHCl₃).

### (EXAMPLES 2 to 4)

Optically active thiazoline compounds were each prepared with the same catalyst and experimental procedure as in EXAMPLE 1, except that ethyl 2-phenylthiazoline-4-carboxylate shown in Table 1 and represented by the following formula was used instead of *tert*-butyl 2-phenylthiazoline-4-carboxylate, and that the compound R³L shown in Table 1 was used instead of methyl iodide.

Table 1 summarizes the reaction time, yield, and optical purity.

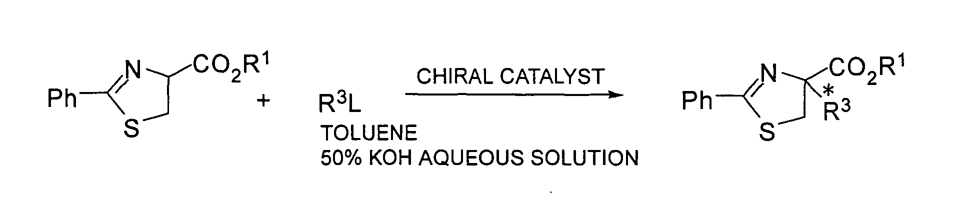

**Table 1**

| | R¹ | R³L | Time (hour) | Yield (%) | % e.e. |
|---|---|---|---|---|---|
| EXAMPLE 2 | -C₂H₅ | C₆H₅CH₂Br | 1.5 | 97 | 97 |
| EXAMPLE 3 | -C₂H₅ | CH₂=CHCH₂Br | 1.5 | 99 | 96 |
| EXAMPLE 4 | -C₂H₅ | HC≡CCH₂Br | 4 | 74 | 95 |

The analytical conditions of NMR spectra and HPLC for the resulting optically active thiazoline compounds are described below. The term "CHIRALPAK AS" refers to a column manufactured by Daicel Chemical Industries, Ltd.

### (EXAMPLE 2) Optically active ethyl 4-benzyl-2-phenylthiazoline-4-carboxylate

¹H NMR (400 MHz, CDCl₃) δ: 7.85-7.87 (2H, m), 7.39-7.50 (3H, m), 7.21-7.28 (5H, m), 4.24 (2H, q), 3.82 (1H, d), 3.43 (1H, d), 3.35 (1H, d), 3.30 (1H, d), 1.27 (3H, t); [α]^{D}₂₇ = +73.6 (c 0.50, CHCl₃); HPLC: CHIRALPAK AS (hexane:ethanol = 200:1), retention time: 11.8 minutes (minor), 13.3 minutes (major).

### (EXAMPLE 3) Optically active ethyl 4-allyl-2-phenylthiazoline-4-carboxylate

¹H NMR (400 MHz, CDCl₃) δ: 7.85-7.87 (2H, m), 7.39-7.49 (3H, m), 5.78-5.85 (1H, m), 5.15-5.21 (2H, m), 4.23-4.31 (2H, m), 3.87 (1H, d), 3.39 (1H, d), 2.76-2.79 (2H, m), 1.31 (3H, t); [α]^{D}₂₇ = +27.8 (c 0.50, CHCl₃); HPLC: CHIRALPAK AS (hexane:ethanol = 200:1), retention time: 9.3 minutes (minor), 10.1 minutes (major).

### (EXAMPLE 4) Optimally active ethyl 4-propargyl-2-phenylthiazoline-4-carboxylate

¹H NMR (400 MHz, CDCl₃) δ: 7.84-7.86 (2H, m), 7.38-7.50 (3H, m), 4.24-4.38 (2H, m), 4.03 (1H, d), 3.62 (1H, d), 3.02 (1H, dd), 2.80 (1H, dd), 2.05 (1H, t), 1.33 (3H, t); [α]^{D}₂₇ = +88.9 (c 0.50, CHCl₃); HPLC: CHIRALCEL OD (hexane:ethanol = 50:1), retention time: 12.5 minutes (minor), 18.6 minutes (major).

### (EXAMPLE 5) Recovery and reuse of catalyst in process for producing optically active tert-butyl 4-methyl-2-phenylthiazoline-4-carboxylate

The same catalyst and experimental procedure as in EXAMPLE 1 were used. After the confirmation of the disappearance of the material by TLC, water was added. The resulting mixture was neutralized with a 1 N hydrobromic acid aqueous solution, and then the organic layer was extracted with methylene chloride. The extracted organic layer was dried over anhydrous sodium sulfate and concentrated to yield a crude product. The crude product was purified by silica-gel column chromatography (hexane:diethyl ether = 10:1) to yield a target product (yield: 86%, 97% e.e.). Next, the catalyst was eluted and recovered from the column with a mixed solvent of methylene chloride and methanol (methylene chloride:methanol = 30:1 to 10:1) (recovery rate: 90%). The recovered catalyst was used again (second cycle), and a target optically active *tert*-butyl 4-methyl-2-phenylthiazoline-4-carboxylate was obtained as a result in 85% yield and 97% e.e. The catalyst was recovered once again and used (third cycle). A target product was obtained in 83% yield and 97% e.e.

### (EXAMPLE 6) Process for producing (R)-α-methyl-L-cysteine hydrochloride

In a nitrogen atmosphere, *tert*-butyl (*R*)-4-methyl-2-phenylthiazoline-4-carboxylate (97% e.e.) (1 g, 3.6 mmol) prepared in EXAMPLE 1 and a 4 N hydrochloric acid aqueous solution (10 g) were added to a glass reactor, and the resulting mixture was refluxed. After the confirmation of the disappearance of *tert*-butyl (R)-4-methyl-2-phenylthiazoline-4-carboxylate by TLC, the resulting mixture was concentrated under a reduced pressure until the total volume was reduced to about 1/6. Toluene (5 mL) was added, and water was azeotropically removed. Furthermore, the azeotropic removal procedure was performed twice with the same amount of toluene used each time. The resulting white crystals were filtrated off, washed with toluene, and dried under a reduced pressure overnight. As a result of the ¹H NMR analysis of the resulting white crystals (0.54 g, yield: 88.0%), the resulting crystals were identified as target (*R*)-α-methyl-L-cysteine hydrochloride.
¹H NMR (300 MHz, D₂O) δ: 3.18 (1H, d), 2.89 (1H, d), 1.60 (3H, s).

### (EXAMPLE 7) Process for determining optical purity of optically active α-methyl-L-cysteine hydrochloride

(*R*)-α-Methyl-L-cysteine hydrochloride (74.9 mg, 0.44 mmol) prepared by a process described in EXAMPLE 6 was dissolved in water (3 mL). To the resulting solution, sodium hydrogencarbonate (197.7 mg) and then 3 mL of ethanol were added. After nitrogen purge, benzyl chlorocarbonate (0.17 mL, 1.10 mmol) was added and the mixture was stirred at room temperature for two days. Concentrated hydrochloric acid was added to the reaction mixture to adjust the pH to 1.9. Extraction was performed with ethyl acetate, and then the resulting organic layer was dried over anhydrous sodium sulfate. The solvent was distilled off under reduced pressure. The resulting mixture was purified by preparative thin-layer chromatography (PTLC, 1:1 hexane/ethyl acetate containing a small amount of acetic acid). As a result of ¹H NMR analysis, the purified compound was identified as target *N,S*-dicarbobenzyloxy-α-methyl-L-cysteine (106 mg, yield: 60%). This compound was analyzed by HPLC (column: CHIRALCEL OD-RH (manufactured by Daicel Chemical Industries, Ltd.), mobile phase: a potassium dihydrogen phosphate·phosphoric acid (pH 2.0) aqueous solution/acetonitrile = 6/4, flow rate: 1.0 mL/min, detection wavelength: 210 nm, column temperature: 30°C, retention time: 19.15 minutes (D isomer), 22.92 minutes (L isomer)). As a result, the optical purity was 97% e.e.
¹H NMR (300 MHz, D₂O) δ: 7.30-7.40 (m, 10H), 5.22 (s, 2H), 5.10 (s, 2H), 3.60 (s, 2H), 1.63 (s, 3H).

### Industrial Applicability

The present invention provides a simple and industrially advantageous process for producing an optically active α-substituted cysteine or a salt thereof, which is useful as an intermediates for pharmaceuticals, from inexpensive and readily available materials with high efficiency, by subjecting a thiazoline compound to a stereoselective substituent-introducing reaction catalyzed by an optically active quaternary ammonium salt, in particular, an axially asymmetric quaternary ammonium salt to produce an optically active thiazoline compound and then hydrolyzing the resulting thiazoline compound.

## Claims

1. A process for producing an optically active thiazoline compound represented by general formula (1): 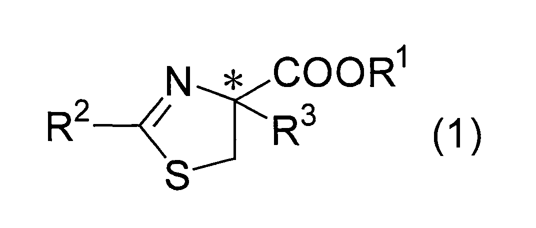 (where * represents an asymmetric carbon atom; R¹ represents an optionally substituted linear, branched, or cyclic C₁-C₁₀ alkyl group or an optionally substituted linear, branched, or cyclic C₁-C₁₀ alkylsilyl group; R² represents an optionally substituted C₆-C₃₀ aryl group or an optionally substituted linear, branched, or cyclic C₁-C₂₀ alkyl group; and R³ represents an optionally substituted linear, branched, or cyclic C₁-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group), the process comprising a step of allowing a thiazoline compound represented by general formula (2): 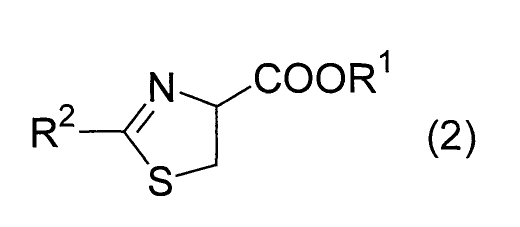 (where R¹ and R² are the same as above) to react with a compound represented by general formula (3) in the presence of a base and an optically active quaternary ammonium salt functioning as a catalyst:
R³L (3)
(R³ is the same as above; and L represents a leaving group).

2. The process according to Claim 1, wherein the optically active quaternary ammonium salt is an optically active axially asymmetric quaternary ammonium salt represented by general formula (4) or general formula (5): 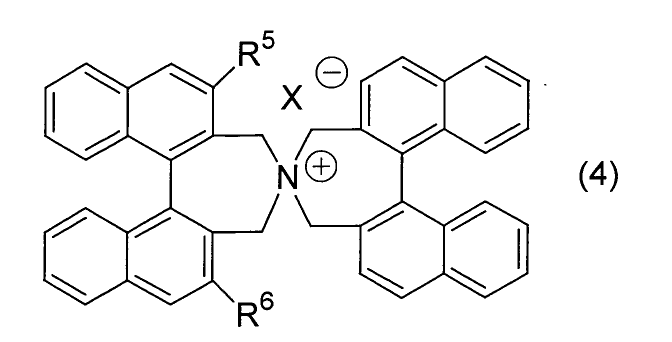 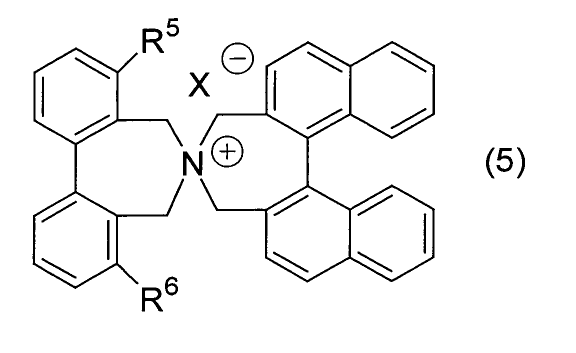 (where R⁵ and R⁶ each represent a hydrogen atom, an optionally substituted linear, branched, or cyclic C₁-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted C₆-C₃₀ aryl group, an optionally substituted C₃-C₃₀ heteroaryl group, an optionally substituted C₇-C₃₀ aralkyl group, an optionally substituted C₄-C₃₀ heteroaralkyl group, an optionally substituted linear, branched, or cyclic C₁-C₁₅ alkanoyl group, or a C₇-C₃₀ aroyl group having an optionally substituted aromatic ring, and R⁵ and R⁶ may be the same or different; and X represents a hetero atom or atomic group having ability to function as a counter anion to the ammonium cation.)

3. The process according to Claim 2, further comprising steps of, after the reaction, isolating and recovering the optically active axially asymmetric quaternary ammonium salt represented by formula (4) or (5) from the reaction mixture by column chromatography using a column packed with an adsorbent, and then reusing the recovered salt.

4. The process according to Claim 2 or 3, wherein R⁵ and R⁶ in formulae (4) and (5) each represent an optionally substituted phenyl group, an optionally substituted naphthyl group, an optionally substituted anthryl group, an optionally substituted phenanthryl group, or an optionally substituted terphenyl group.

5. The process according to any one of Claims 2 to 4, wherein R⁵ and R⁶ in formulae (4) and (5) represent the same group.

6. The process according to any one of Claims 2 to 5, wherein, in formulae (4) and (5), each X represents a halogen atom.

7. The process according to any one of Claims 1 to 6, wherein R¹ represents a methyl group, an ethyl group, an *n*-propyl group, an isopropyl group, an *n*-butyl group, a *sec*-butyl group, or a *tert*-butyl group.

8. The process according to any one of Claims 1 to 7, wherein R² represents an optionally substituted phenyl group.

9. The process according to any one of Claims 1 to 8, wherein R³ represents a methyl group, an ethyl group, an allyl group, a propargyl group, or a benzyl group.

10. The process according to any one of Claims 1 to 9, wherein L in formula (3) represents a halogen atom.

11. A process for producing an optically active α-substituted cysteine represented by general formula (6) or a salt thereof: 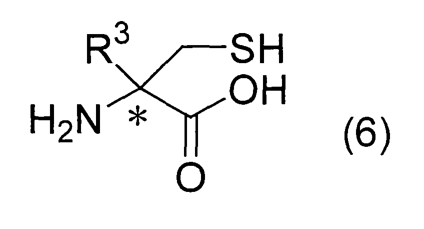 (where * represents an asymmetric carbon atom; and R³ represents an optionally substituted linear, branched, or cyclic C₁-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group), the process comprising a step of hydrolyzing an optically active thiazoline compound produced by the process according to any one of Claims 1 to 10, the thiazoline compound being represented by general formula (1): 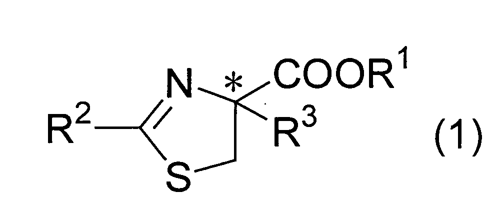 (where * and R³ are the same as above; R¹ represents an optionally substituted linear, branched, or cyclic C₁-C₁₀ alkyl group or an optionally substituted linear, branched, or cyclic C₁-C₁₀ alkylsilyl group; and R² represents an optionally substituted C₆-C₃₀ aryl group or an optionally substituted linear, branched, or cyclic C₁-C₂₀ alkyl group).

12. A process for producing an optically active α-substituted cysteine represented by general formula (6) or a salt thereof: 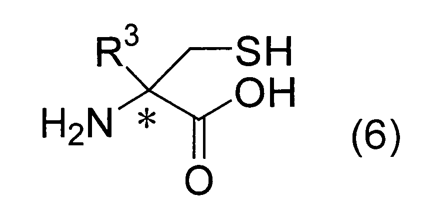 (where * represents an asymmetric carbon atom; and R³ represents an optionally substituted linear, branched, or cyclic C₁-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group), the process comprising a step of hydrolyzing an optically active thiazoline compound represented by general formula (1): 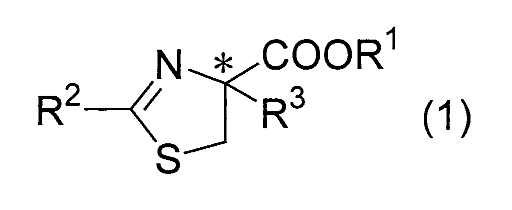 (where * and R³ are the same as above; R¹ represents an optionally substituted linear, branched, or cyclic C₁-C₁₀ alkyl group or an optionally substituted linear, branched, or cyclic C₁-C₁₀ alkylsilyl group; and R² represents an optionally substituted C₆-C₃₀ aryl group or an optionally substituted linear, branched, or cyclic C₁-C₂₀ alkyl group).

13. The process according to Claim 11 or 12, wherein an acid is used for the hydrolysis.

14. An optically active thiazoline compound represented by general formula (7): 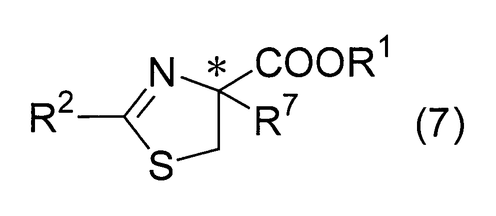 (where * represents an asymmetric carbon atom; R¹ represents an optionally substituted linear, branched, or cyclic C₁-C₁₀ alkyl group or an optionally substituted linear, branched, or cyclic C₁-C₁₀ alkylsilyl group; R² represents an optionally substituted C₆-C₃₀ aryl group or an optionally substituted linear, branched, or cyclic C₁-C₂₀ alkyl group; and R⁷ represents an optionally substituted linear, branched, or cyclic C₂-C₂₀ alkyl group, an optionally substituted linear, branched, or cyclic C₂-C₂₀ alkenyl group, an optionally substituted linear, branched, or cyclic C₂-C₂₀ alkynyl group, an optionally substituted linear, branched, or cyclic C₃-C₂₀ alkoxycarbonylalkyl group, an optionally substituted C₇-C₃₀ aralkyl group, or an optionally substituted C₄-C₃₀ heteroaralkyl group).

15. The compound according to Claim 14, wherein R¹ represents a methyl group, an ethyl group, an *n*-propyl group, an isopropyl group, an *n*-butyl group, a *sec*-butyl group, or a *tert*-butyl group.

16. The compound according to Claim 14 or 15, wherein R² represents an optionally substituted phenyl group.

17. The compound according to Claim 14, 15, or 16, wherein R⁷ represents an ethyl group, an *n*-propyl group, an isopropyl group, an *n*-butyl group, a *sec*-butyl group, a *tert*-butyl group, a pentyl group, a hexyl group, a cyclopropylmethyl group, a cyclopentylmethyl group, a cyclohexylmethyl group, an allyl group, a 2-butenyl group, a 1-methyl-2-propenyl group, a 2-methyl-2-propenyl group, a propargyl group, a *tert*-butoxycarbonylmethyl group, a benzyl group, a chlorobenzyl group, a fluorobenzyl group, a bromobenzyl group, a dichlorobenzyl group, a difluorobenzyl group, a dibromobenzyl group, a methylbenzyl group, a methoxybenzyl group, a 3,4-dibutoxybenzyl group, a naphthylmethyl group, or an indolylmethyl group.
